**Europäisches Patentamt**

**European Patent Office**

**Office européen des brevets**

(19)

(11) Veröffentlichungsnummer : **0 595 766 A1**

(12)

# EUROPÄISCHE PATENTANMELDUNG

(21) Anmeldenummer : **93810720.8**

(22) Anmeldetag : **13.10.93**

(51) Int. Cl.$^5$ : **A61K 31/195,** A61K 47/10, A61K 47/18

(30) Priorität : **22.10.92 CH 3275/92**

(43) Veröffentlichungstag der Anmeldung :
**04.05.94 Patentblatt 94/18**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Galli, Bruno, Dr.**
**Liestalerstrasse 28 D**
**CH-4411 Seltisberg (CH)**

(54) **Parenterale Lösungen für Diclofenacsalze.**

(57)  Gegenstand der Erfindung ist eine pharmazeutische Zusammensetzung in Form einer sterilisierbaren parenteralen Lösung enthaltend ein Diclofenacsalz und Stabilisatoren wie Milchsäureäthylester kombiniert mit Glutathion oder N-Acetylcystein.

EP 0 595 766 A1

Gegenstand der Erfindung ist eine pharmazeutische Zusammensetzung in Form einer sterilisierbaren parenteralen Lösung enthaltend ein Diclofenacsalz und Stabilisatoren, Verfahren zur Herstellung dieser Lösung sowie die Verwendung von Stabilisatoren in diesem Herstellungsverfahren.

Für die Behandlung von entzündlichen Krankheiten, z.B. Rheumatismus, stehen verschiedene Arzneimittel mit unterschiedlicher Struktur zur Verfügung. Da solche Krankheiten oft chronisch verlaufen, muss die Behandlung mit entzündungshemmenden Arzneimitteln (Antiinflammatorika/Antiphlogistika) in der Regel über einen längeren Zeitraum mit regelmässiger Wirkstoffzufuhr ohne Unterbrechungen erfolgen. Insbesondere können manche nichtsteroidale Antiinflammatorika (NSAID) bei lang andauernder peroraler Verabreichung Nebenwirkungen im gesamten gastrointestinalen Trakt, z.B. Nausea, Erbrechen, epigastrische Schmerzen und auch Ulcus ventriculi, hervorrufen. Ausserdem erfolgt die Freisetzung des Wirkstoffs aus peroralen Darreichungsformen wie Tabletten oder Dragees nur langsam, was bei der Behandlung von plötzlich akut auftretenden Rheumabeschwerden nachteilig sein kann.

Zur Gruppe der NSAID der ersten Wahl zählt das seit langem in zahlreichen Ländern eingeführte Natriumsalz von Diclofenac, das in verschiedenen Darreichungsformen, wie Dragées, Suppositorien oder Injektionslösungen, unter dem Warenzeichen ®Voltaren (Ciba-Geigy) erhältlich ist.

Von Diclofenacsalzen ist bekannt, dass geringe Mengen in wässriger Lösung, insbesondere bei erhöhter Temperatur unter den Bedingungen der Hitzesterilisation, zu 1-(2,6-Dichlorphenyl)-indolin-2-on unter Abspaltung von Hydroxidionen cyclisieren können. Durch Oxydation mit in der Lösung vorhandenem Sauerstoff können sich weitere unerwünschte Folgeprodukte bilden.

Zur Unterdrückung von Oxydationsreaktionen setzt man Injektionslösungen Antioxidantien u.a. aus der Gruppe der reduzierenden Schwefelverbindungen hinzu, siehe Pharmazeutische Technologie, herausgegeben von Heinz Sucker et al., G.Thieme Verlag Stuttgart DE, 1978, Seite 313. So enthält die kommerziell erhältliche Injektionslösung mit dem Wirkstoff VOLTAREN, siehe **Rote Liste** 1987, Nr.05169, Verzeichnis von Fertigarzneimitteln, Editio Cantor, den als Antioxidans für Injektabilia zugelassenen Hilfsstoff Natriumdisulfit (Natriumpyrosulfit). Reduktionsreaktionen und/oder Zerfallsreaktionen dieser Hilfsstoffe können pH-Änderungen der wässrigen Lösung ausserhalb des physiologisch akzeptablen Bereichs von 7 bis 8 bewirken. Die zu sterilisierende Injektionslösung muss daher nach Zugabe des Antioxidans in einem Folgeschritt neutralisiert oder abgepuffert werden, um die Forderung nach physiologisch annehmbaren pH-Bedingungen für die parenteral, insbesondere i.m., zu applizierende Lösung zu erfüllen.

Von Antioxidantien aus der Gruppe der reduzierenden Schwefelverbindungen ist deren allergenes Potential mit Risiko von Asthmaanfällen und anaphylaktischen Schocks bekannt, siehe der Übersichtsartikel : Sulfite Hypersensivity von A.F.Gunnison und D.W. Jacobsen in CRC Critical Reviews in Toxicology, Vol.17 (1987), Issue 3, Seiten 185-214, siehe insbesondere Abschnitt C, Seiten 195-197. Der Zusatz von Sulfiten zu Fertigarzneimitteln muss daher deklariert werden und ein Warnhinweis auf der Packung erfolgen (Proposal U.S. Fed.Reg. 19.11.1985).

Die der vorliegenden Erfindung zugrunde liegende Aufgabe besteht in der Herstellung einer neuen, verbesserten sulfitfreien Darreichungsform für die parenterale Applikation von Diclofenac-Salzen, welche gegenüber den bisher bekannten hitzesterilisierbaren Injektionslösungen sowohl den Vorteil der Stabilität des pH-Werts als auch der verbesserten Verträglichkeit des verwendeten Antioxidans, verbunden mit raschem Wirkungseintritt und lang andauernder Wirkung des Wirkstoffs auf therapeutischem Niveau, aufweist.

Diese Aufgabe wird durch die vorliegende Erfindung gelöst, welche eine pharmazeutische Zusammensetzung in Form einer parenteral applizierbaren Injektionslösung mit dem Wirkstoff Diclofenac bzw. einem pharmazeutisch annehmbaren Salz davon betrifft. Diese Injektionslösung enthält:

a) ein pharmazeutisch annehmbares Salz von Diclofenac;

b) 1,2-Propylenglycol oder Polyethylenglycol 300 - 400 als Löslichkeitsvermittler;

c) einen pharmazeutisch annehmbaren Stabilisator ausgewählt aus der Gruppe Glutathion, Glutathion kombiniert mit $C_{2-3}$-Alkancarbonsäure-$C_{2-4}$-alkylester oder mit Hydroxy-$C_{2-4}$-alkancarbonsäure-$C_{2-4}$-alkylester oder mit N-Acetylcystein und N-Acetylcystein kombiniert mit $C_{2-3}$-Alkancarbonsäure-$C_{2-4}$-alkylester oder mit Hydroxy-$C_{2-4}$-alkancarbonsäure-$C_{2-4}$-alkylester und

d) eine für Injektionszubereitungen verwendbare Trägerflüssigkeit und gegebenenfalls weitere dafür verwendbare Hilfsstoffe.

Bei Verwendung der Stabilisatoren der Komponente c) wird keine Bildung von Oxydationsprodukten der Indolin-2-on-Verbindung über die zulässigen Grenzwerte hinaus festgestellt. Ebenso wird eine unerwünschte Bildung von Oxydationsprodukten des Diclofenac-Salzes wirksam unterbunden. Ausserdem bleibt der pH-Wert der Lösung über einen langen Zeitraum bis hin zu mehreren Monaten konstant neutral.

Die weiter vorn und im folgenden genannten Begriffe und Definitionen haben im Rahmen der Beschreibung der Erfindung vorzugsweise die folgenden Bedeutungen:

Der Begriff pharmazeutische Zusammensetzung definiert eine Lösung, welche sich zur parenteralen Applikation, insbesondere i.m. aber auch i.v., eignet und zur Behandlung von entzündlichen Zuständen verbunden mit Schmerzen, insbesondere Rheuma, verwendbar ist.

Komponente a) - Ein pharmazeutisch annehmbares Salz von Diclofenac, o-(2,6- Dichlorophenylamino)-phenylessigsäure, ist insbesondere ein Alkalimetallsalz, z.B. das Natrium- oder das Kaliumsalz, oder das mit einem Amin gebildete Salz, z.B. einem Mono-, Di- oder Tri-$C_1$-$C_4$-alkylamin, z.B. Diäthyl- oder Triäthylamin, Hydroxy-$C_2$-$C_4$-alkylamin, z.B. Äthanolamin, Hydroxy-$C_2$-$C_4$-alkyl-$C_1$-$C_4$-alkylamin, z.B. Dimethyläthanolamin, oder ein quaternäres Ammoniumsalz, z.B. das Tetramethylammoniumsalz oder das Cholinsalz von Diclofenac.

Besonders bevorzugt sind das Natrium- und das Kaliumsalz von Diclofenac, siehe Merck Index, Eleventh Edition No. 3071. Diese Salze sind in der Injektionslösung in einer Dosierung von ca. 25 mg bis ca. 150 mg enthalten. Die bevorzugte Wirkstoffkonzentration in der pharmazeutischen Zusammensetzung beträgt ca. 10 mg/ml bis ca. 50 mg/ml.

Komponente b) - Der Löslichkeitsvermittler ist 1,2-Propylenglycol (1,2-Propanediol, Merck Index Eleventh Edition No.7868) oder Polyethylenglycol mit einer Molmasse von ca. 300 - 400 (Merck Index Eleventh Edition No. 7545). Diese Löslichkeitsvermittler sind in der pharmazeutischen Zusammensetzung mit einem Gehalt von ca. 5% bis ca. 50% (Gew./Vol.), vorzugsweise von ca. 20% bis ca. 35%, enthalten. Die bevorzugten Mengen dieser Komponente betragen pro Formulierungen 500 - 2000mg, insbesondere 500 - 1000 mg.

Komponente c) - Der pharmazeutisch annehmbare Stabilisator $C_{2-3}$-Alkancarbonsäure-$C_{2-4}$-alkylester ist z.B. der Essigsäure- oder Propionsäureäthylester. Der Hydroxy-$C_{2-4}$-alkancarbonsäure-$C_{2-4}$-alkylester ist insbesondere der Äthylester der Milchsäure. Als Stabilisatoren können racemische Gemische oder optisch reine Formen (Enantiomere) der Milchsäure verwendet werden. In einer bevorzugten Ausführungsform wird Milchsäureäthylester in Kombination mit Glutathion oder mit N-Acetylcystein als Stabilisator der injizierbaren pharmazeutischen Zusammensetzung verwendet. Es ist auch bisher nicht bekannt, dass diese Kombination von Hilfsstoffen den pH-Wert von wässrigen Lösungen enthaltend Diclofenac-Salze bei neutralen oder annähernd neutralen Bedingungen wirksam stabilisieren.

Glutathion, siehe Merck Index Eleventh Edition Nr. 4369 ist als pharmazeutischer Hilfsstoff bekannt, siehe R.Voigt, Lehrbuch der Pharmazeutischen Chemie, 6.Auflage, Verlag Chemie Weinheim, Bundesrepublik Deutschland, Seiten 504-506, worin die Verwendung dieses Hilfsstoffs als Antioxidans für hydrophile Systeme beschrieben ist.

N-Acetyl-L-cystein, siehe Merck Index Eleventh Edition Nr 82, ist als pharmazeutischer Hilfsstoff bekannt. Glutathion oder N-Acetylcystein ist in einer bevorzugten Menge von ca. 1,0 - 5,0 mg pro Formulierung vorhanden. Kombiniert mit diesen Stabilisatoren wird Ethyllactat in einer bevorzugten Menge von ca. 0,02 - 3,0 mg hinzugesetzt.

Die Komponente c) ist in der pharmazeutischen Zusammensetzung insgesamt mit einem Gehalt von ca. 0,1 mg/ml bis ca. 3 mg/ml, vorzugsweise ca. 1 mg/ml bis ca. 2 mg/ml,vorhanden.

Die pharmazeutisch annehmbare Trägerflüssigkeit d) ist Wasser, das nach den Vorschriften der nationalen Pharmakopöen für intravenöse Lösungen keim- und pyrogenfrei gemacht wurde.

In der Trägerflüssigkeit d) können für Injektionszubereitungen verwendbare, nicht toxische Hilfsstoffe enthalten sein, z.B. wasserlösliche Hilfsstoffe welche zur Herstellung von isotonischen Bedingungen erforderlich sind, z.B. ionische Zusätze wie Kochsalz oder nichtionische Zusätze wie Sorbit, Mannit, Glucose, Lactose, Fructose oder Sucrose. Insbesondere sind diese Zusätze, z.B. Kochsalz oder Mannit, in den in nationalen Pharmakopöen vorgeschriebenen Mengen enthalten, welche zur Einstellung von isotonischen Bedingungen der Injektionslösungen erforderlich sind.

Das Volumen der Trägerflüssigkeit d) beträgt im Gemisch mit den Komponenten a), b) und c) beispielweise ein Volumen von ca. 1-5 ml, vorzugsweise 1-2 ml. In bevorzugten Ausführungsformen haben die Injektionslösungen, die die üblichen Dosen von 50, 75 oder 100 mg Diclofenac-Natrium enthalten, ein Gesamtvolumen von ca. 1-5 ml, vorzugsweise 3 ml.

Die pharmazeutische Zusammensetzung gemäss vorliegender Erfindung ist als Injektionslösung für die parenterale Applikation, insbesondere i.m. und i.v., zur Behandlung von Schmerzzuständen, Entzündungen und/oder rheumatischen Erkrankungen bei Warmblütern (Menschen) verwendbar. Es können Tagesdosen von ca. 25 bis 200 mg Wirkstoff appliziert werden, wobei die einzelne Darreichungsform die übliche Wirkstoffmenge von beispielsweise 25, 50, 75, 100 oder 150 mg enthält.

Die Erfindung hat ebenfalls das Herstellungsverfahren für die pharmazeutische Zusammensetzung zum Gegenstand. Dieses Verfahren ist dadurch gekennzeichnet, dass man in der Trägerflüssigkeit d) die Komponenten a), b) und c) in beliebiger Reihenfolge löst und gegebenenfalls weitere für Injektionszubereitungen verwendbare Hilfsstoffe zusetzt.

In einer bevorzugten Ausführungsform des Verfahrens setzt man zur Trägerflüssigkeit d) die Komponenten a) - Wirkstoff - und b) - Löslichkeitsvermittler - zu. Zu dieser Lösung gibt man anschliessend die Komponente c) - Stabilisatoren -.

Der Wirkstoff a) wird vorzugsweise in zerkleinerter Form zu einer vorgelegten Teilmenge der Trägerflüssigkeit d), vorzugsweise in fein gemahlener Form, hinzugefügt. Fein gemahlenes Diclofenacsalz hat eine bevorzugte durchschnittliche Teilchengrösse kleiner als 200 μm und ist insbesondere mit einer Teilchengrösse kleiner als 100 μm verwendbar. Zur Herstellung von Partikeln mit dieser Teilchengrösse bedient man sich der üblichen Zerkleinerungstechniken, z.B. Mahlen in einer Luftstrahl-, Kugel- oder Vibratormühle oder nach an sich bekannten Verfahren in einem Ultraschalldesintegrator, z.B. vom Typ Branson Sonifier, z.B. wie in J. Pharm. Sci. 53 (9), 1040-1045 (1965), beschrieben, oder mittels hochtourigem Rühren einer Suspension, z.B. mit einem Rührer des Typs Homorex der Fa. Brogli & Co. Basel.

Die vorzulegende Teilmenge der Trägerflüssigkeit d) beträgt ca. 1-2 ml, der man die Gesamtmenge des Löslichkeitsvermittlers b) und den fein gemahlenen Wirkstoff a) sowie die Stabilisatoren c) zufügt.

Man macht beispielsweise die leicht sauer reagierende wässrige Lösung bis zu einem pH von ca. 8,0 basisch. Dies kann durch Zugabe einer pharmazeutisch annehmbaren, verdünnten wässrigen Base erfolgen, z.B. verdünnter Natronlauge, 0,1 N NaOH-Lösung bevorzugt. Man arbeitet üblicherweise unter gleichzeitiger pH-Kontrolle. Gegebenenfalls ergänzt man mit sterilem, keimfreien und pyrogenfreien Wasser auf das erforderliche Injektionsvolumen. Die Injektionslösung lässt sich in Ampullen oder Injektionsfläschchen (Vials) mit geeigneten Volumina unter aseptischen Bedingungen abfüllen und im Autoklaven hitzesterilisieren (oberhalb 120°/mindestens 15 min.)

Ebenfalls Gegenstand der Erfindung ist die Verwendung des Stabilisators c) ausgewählt aus der Gruppe Glutathion, Glutathion kombiniert mit $C_{2-3}$-Alkancarbonsäure-$C_{2-4}$-alkylester oder mit Hydroxy-$C_{2-4}$-alkancarbonsäure-$C_{2-4}$-alkylester oder mit N-Acetylcystein und N-Acetylcystein kombiniert mit $C_{2-3}$-Alkancarbonsäure-$C_{2-4}$-alkylester oder mit Hydroxy-$C_{2-4}$-alkancarbonsäure-$C_{2-4}$-alkylester in dem weiter vorn beschriebenen Verfahren.

Die vorliegende Erfindung betrifft bevorzugt eine pharmazeutische Zusammensetzung in Form einer parenteral applizierbaren Injektionslösung enthaltend:

a) das Natriumsalz von Diclofenac;

b) 1,2-Propylenglycol oder Polyethylenglycol 300 als Löslichkeitsvermittler;

c) Milchsäureäthylester kombiniert mit Glutathion oder N-Acetylcystein als Stabilisatoren und

d) eine für Injektionszubereitungen verwendbare Trägerflüssigkeit und gegebenenfalls weitere dafür verwendbare Hilfsstoffe.

Die Erfindung betrifft insbesondere auch die in den Beispielen beschriebenen Zubereitungen.

Die folgenden Beispiele dienen der Erläuterung der weiter vorn vollständig beschriebenen Erfindung; sie sollen diese in ihrem Umfang keinesfalls einschränken.

| Beispiel 1 | |
|---|---|
| Diclofenac-Natrium (®Voltaren) | 75,0 mg |
| 1,2-Propylenglycol (dest.) | 1000,0 mg |
| Milchsäureäthylester | 0,1 mg |
| Glutathion oder N-Acetylcystein | 2,0 mg |
| 1 N NaOH-Lösung | bis pH 8,3 |
| Wasser pro inj. | ad 3,0 ml |

Unter Stickstoff-Schutzgasatmosphäre wird ein Teil des Wassers vorgelegt. Darin löst man die Gesamtmenge Glutathion oder N-Acetylcystein. Man fügt die gesamte Menge 1,2-Propylenglycol sowie den Wirkstoff, den man zuvor auf eine durchschnittliche Teilchengrösse von ca.100 μm gemahlen hat, hinzu. Mit 0,1 N Natronlaugelösung stellt man den pH-Wert der Lösung auf pH 8,3 und setzt Milchsäureäthylester hinzu. Nach Auffüllen mit Wasser auf 3 ml (pro Formulierung) wird die Lösung mit einem Sterilfilter mit 0,2 μm Porenweite (bestehend z.B. aus NYLON, Polypropylen oder Acrylcopolymer) filtriert und anschliessend 15 Minuten lang bei 121°C sterilisiert. Man füllt in Brechampullen jeweils 3,3 ml der Lösung aus dem Gesamtansatz ab.

Beispiel 2

| Diclofenac-Natrium (®Voltaren) | 75,0 mg |
| Polyethylenglycol 300 | 1000,0 mg |
| Glutathion oder N-Acetylcystein | 2,0 mg |
| Ethyllactat | 0,1 mg |
| 1 N NaOH-Lösung | bis pH 8,0 |

Wasser pro inj.                                      ad 3,0 ml

Unter Stickstoff-Schutzgasatmosphäre wird ein Teil des Wassers vorgelegt. Darin löst man die Gesamtmenge Glutathion oder N-Acetylcystein kombiniert mit Ethyllactat. Man fügt die gesamte Menge Polyethylenglycol 300 sowie den Wirkstoff, den man zuvor auf eine durchschnittliche Teilchengrösse von ca.100 µm gemahlen hat, hinzu. Mit 0,1 N Natronlaugelösung stellt man den pH-Wert der Lösung auf pH 8,0 . Nach Auffüllen mit Wasser auf 3 ml (pro Formulierung) wird mit einem Sterilfilter mit 0,2 µm Porenweite (bestehend z.B. aus NYLON, Polypropylen oder Acrylcopolymer) filtriert und anschliessend 15 Minuten lang bei 121°C sterilisiert. Man füllt in Brechampullen jeweils 3,3 ml der Lösung aus dem Gesamtansatz ab.

| Beispiel 3 (analog Beispiel 1 oder 2) | |
|---|---|
| Diclofenac-Natrium (®Voltaren) | 75,0 mg |
| Polyethylenglycol 300 | 780 mg |
| Benzylalkohol | 120 mg |
| Mannit | 8,6 mg |
| Ethyllactat | 0,1 mg |
| Glutathion oder N-Acetylcystein | 2,0 mg |
| 1 N NaOH-Lösung | bis pH 8,0 |
| Wasser pro inj. | ad 2,0 ml oder |
| | ad 3,0 ml |

| Beispiel 4 (analog Beispiel 1 oder 2) | |
|---|---|
| Diclofenac-Natrium (®Voltaren) | 75,0 mg |
| 1,2-Propylenglycol | 780 mg |
| Benzylalkohol | 120 mg |
| Mannit | 8,6 mg |
| Ethyllactat | 0,1 mg |
| Glutathion oder N-Acetylcystein | 2,0 mg |
| 1 N NaOH-Lösung | bis pH 8,0 |
| Wasser pro inj. | ad 2,0 ml oder |
| | ad 3,0 ml |

**Patentansprüche**

1. Pharmazeutische Zusammensetzung in Form einer parenteral applizierbaren Injektionslösung enthaltend:
   a) ein pharmazeutisch annehmbares Salz von Diclofenac;
   b) 1,2-Propylenglycol oder Polyethylenglycol 300 - 400 als Löslichkeitsvermittler;
   c) einen pharmazeutisch annehmbaren Stabilisator ausgewählt aus der Gruppe Glutathion, Glutathion kombiniert mit $C_{2-3}$-Alkancarbonsäure-$C_{2-4}$-alkylester oder mit Hydroxy-$C_{2-4}$-alkancarbonsäure-$C_{2-4}$-alkylester oder mit N-Acetylcystein und N-Acetylcystein kombiniert mit $C_{2-3}$-Alkancarbonsäure-$C_{2-4}$-alkylester oder mit Hydroxy-$C_{2-4}$-alkancarbonsäure-$C_{2-4}$-alkylester und
   d) eine für Injektionszubereitungen verwendbare Trägerflüssigkeit und gegebenenfalls weitere dafür verwendbare Hilfsstoffe.

2. Pharmazeutische Zusammensetzung gemäss Anspruch 1 enthaltend:
   a) das Natriumsalz von Diclofenac;
   b) 1,2-Propylenglycol oder Polyethylenglycol 300 als Löslichkeitsvermittler;
   c) Milchsäureäthylester kombiniert mit Glutathion oder mit N-Acetylcystein als Stabilisatoren und
   d) eine für Injektionszubereitungen verwendbare Trägerflüssigkeit und gegebenenfalls weitere dafür verwendbare Hilfsstoffe.

3. Verfahren zur Herstellung einer pharmazeutischen Zusammensetzung gemäss Anspruch 1, dadurch gekennzeichnet, dass man in der Trägerflüssigkeit d) die Komponenten a), b) und c) in beliebiger Reihenfolge löst und gegebenenfalls weitere für Injektionszubereitungen verwendbare Hilfsstoffe zusetzt.

4. Verwendung eines pharmazeutisch annehmbaren Stabilisators ausgewählt aus der Gruppe Glutathion, Glutathion kombiniert mit $C_{2-3}$-Alkancarbonsäure-$C_{2-4}$-alkylester oder mit Hydroxy-$C_{2-4}$-alkancarbonsäure-$C_{2-4}$-alkylester oder mit N-Acetylcystein und N-Acetylcystein kombiniert mit $C_{2-3}$-Alkancarbonsäure-$C_{2-4}$-alkylester oder mit Hydroxy-$C_{2-4}$-alkancarbonsäure-$C_{2-4}$-alkylester zur Herstellung einer pharmazeutischen Zusammensetzung für die parenterale Applikation eines pharmazeutisch annehmbaren Salzes von Diclofenac.

5. Pharmazeutische Zusammensetzung gemäss Anspruch 1 zur Anwendung in einem Verfahren zur therapeutischen Behandlung des menschlichen oder tierischen Körpers.

**Europäisches Patentamt**

# EUROPÄISCHER RECHERCHENBERICHT

Nummer der Anmeldung

EP 93 81 0720

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 185 374 (MERCKLE GMBH) * das ganze Dokument * ----- | 1-5 | A61K31/195 A61K47/10 A61K47/18 |

**RECHERCHIERTE SACHGEBIETE (Int.Cl.5)**

A61K

Der vorliegende Recherchenbericht wurde für alle Patentansprüche erstellt

| Recherchenort | Abschlußdatum der Recherche | Prüfer |
|---|---|---|
| DEN HAAG | 14. Dezember 1993 | Ventura Amat, A |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

........................................................................
& : Mitglied der gleichen Patentfamilie, übereinstimmendes Dokument

EPO FORM 1503 03.82 (P04C00)